(19) Europäisches Patentamt
European Patent Office
Office européen des brevets

(11) EP 0 672 184 B1

(12) **FASCICULE DE BREVET EUROPEEN**

(45) Date de publication et mention de la délivrance du brevet:
**29.12.1997 Bulletin 1997/52**

(21) Numéro de dépôt: **93914800.3**

(22) Date de dépôt: **02.07.1993**

(51) Int Cl.[6]: **C12Q 1/68**

(86) Numéro de dépôt international:
**PCT/FR93/00675**

(87) Numéro de publication internationale:
**WO 94/01581 (20.01.1994 Gazette 1994/03)**

(54) **PROCEDE DE DETERMINATION DE LA TAILLE EN NUCLEOTIDES DE FRAGMENTS D'ADN**

METHODEN ZUR BESTIMMUNG DES NUKLEOTIDGRÖSSE IN DNA-FRAGMENTEN

METHOD FOR DETERMINING THE NUCLEOTIDE SIZE OF DNA FRAGMENTS

(84) Etats contractants désignés:
**AT BE CH DE DK ES FR GB GR IE IT LI LU MC NL PT SE**

(30) Priorité: **03.07.1992 FR 9208253**

(43) Date de publication de la demande:
**20.09.1995 Bulletin 1995/38**

(73) Titulaires:
- **INSTITUT NATIONAL DE LA SANTE ET DE LA RECHERCHE MEDICALE (INSERM) (E.P.S.T.) 75013 Paris (FR)**
- **INSTITUT PASTEUR F-75015 Paris (FR)**

(72) Inventeur: **PANNETIER, Christophe F-75013 Paris (FR)**

(74) Mandataire: **Martin, Jean-Jacques
Cabinet REGIMBEAU
26, Avenue Kléber
75116 Paris (FR)**

(56) Documents cités:
**WO-A-90/04648**
**WO-A-92/00796**
**WO-A-92/12260**
**US-A- 4 811 218**

- **T.MANIATIS ET AL. (EDS.) 'Molecular cloning. A laboratory manual' 1989 , COLD SPRING HARBOR LABORATORY PRESS , NEW YORK US**
- **A.L.LEHNINGER 'Biochemistry' 1975 , WORTH PUBLISHERS, INC. , NEW YORK US**
- **DATABASE WPIL Week 9151, Derwent Publications Ltd., London, GB; AN 91-371721**
- **ANALYTICAL BIOCHEMISTRY vol. 189, no. 2, 1 Septembre 1990, NEW YORK US pages 235 - 243 K.E.OERTER ET AL.**
- **BIOCHEMISTRY vol. 22, no. 26, 1983, EASTON US pages 6180 - 6185 N.C.STELLWAGEN**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 672 184 B1

Printed by Jouve, 75001 PARIS (FR)

## Description

La présente invention concerne un procédé permettant de déterminer la taille en nucléotides de fragments d'ADN.

Le procédé conforme à la présente invention exploite un processus de séparation des fragments d'ADN, par électrophorèse sur gel.

La séparation et la détection de fragments d'ADN marqués, par exemple marqués par des marqueurs fluorescents, par électrophorèse sur gel, sont bien connues de l'homme de l'art.

On connait sur le marché différents appareillages aptes à réaliser ces fonctions. Parmi ces appareillages connus on peut citer le dispositif automatisé d'électrophorèse sur gel à séquençage d'ADN automatique commercialisé par la Société Applied Biosystem sous la référence 373A. Ce dispositif est décrit dans le document US-A-4 811 218.

Les appareillages connus de séparation de fragments d'ADN par électrophorèse sur gel comprennent généralement :

- un support plan de migration formé d'un gel, par exemple en polyacrylamide ou agarose, apte à recevoir des échantillons d'ADN préalablement marqués,
- une source d'alimentation électrique continue haute tension, typiquement de 1000 à 1500V, connectée entre les extrémités du gel support pour induire la séparation des fragments d'ADN, par électrophorèse,
- un ensemble de détection des fragments d'ADN migrant successivement sur le gel support, placé à la base de celui-ci et
- un ensemble d'enregistrement et de traitement des données issues de l'ensemble de détection.

Les échantillons d'ADN sont préparés selon diverses techniques bien connues de l'homme de l'art.

Ces techniques de préparation connues utilisent généralement une méthode d'amplification enzymatique in vitro de séquences d'acides nucléiques ADN mono-brins ou double brins, notamment par PCR (Polymerase Chain Reaction) ou par la technique par SDA (Strand Displacement Amplification).

Les techniques d'amplification in vitro, notamment par PCR ou SDA, ont été décrites dans la littérature. On peut citer en particulier les publications EP-A-201 184 et EP-A-200 362 sur la technique de base pour la méthode PCR. La technique d'amplification dite par SDA (Strand Displacement Amplification) a été décrite lors de la conférence de San Diego sur les Acides Nucléiques les 20-22 novembre 1991.

Dans ces méthodes d'amplification enzymatique d'ADN, l'amplification de la séquence s'effectue par cycles successifs.

Chaque cycle comporte plusieurs étapes :

- une étape d'hybridation d'amorces oligonucléotides spécifiques sur les extrémités 5' des fragments d'ADN qui leur sont complémentaires, et
- une étape d'élongation à partir des extrémités 3' des amorces à l'aide d'une ADN polymérase.

Le facteur de multiplication des fragments d'ADN est en théorie de deux à chaque cycle.

Dans les techniques d'amplification enzymatique d'ADN du type PCR, les échantillons sont dénaturés par la chaleur au début de chaque cycle. L'emploi d'une polymérase thermostable, la Taq polymérase, a permis de développer des cycleurs automatiques ( dénaturation thermique / hybridation / polymérisation enzymatique ) dont les différentes étapes ne se distinguent que par leur température opératoire. Les conditions de dénaturation correspondent généralement à une élévation de la température du milieu réactionnel au-dessus de 90°C, l'hybridation a lieu généralement entre 50 et 70°C et l'élongation par l'ADN polymérase peut s'effectuer à des températures relativement élevées, de l'ordre de 70°C si l'on utilise une ADN polymérase stable à la chaleur.

En revanche, dans d'autres techniques d'amplification enzymatique d'ADN, telles que la technique par SDA, les produits obtenus à la fin de chaque cycle ne sont pas dénaturés par la chaleur. Il s'agit de méthodes isothermes.

La méthode d'amplification par SDA repose sur l'utilisation d'amorces oligonucléotidiques modifiées en 5' par l'addition d'une séquence d'ADN reconnue par une enzyme de restriction, par exemple l'enzyme Hinc II. Le processus requiert la formation d'un site de restriction thiolé par incorporation de déoxyadénosine triphostate soufré, et les actions alternées de ladite enzyme Hinc II qui hydrolyse partiellement (sur un seul brin), ledit site de restriction, et de l'ADN polymérase qui synthétise à partir du point d'hydrolyse un nouveau brin, avec déplacement simultané de la séquence nucléique précédemment coupée, sans que la dénaturation soit nécessaire. L'hybridation des amorces modifiées sur la cible nécessite seulement une première étape de dénaturation de l'ADN. La réaction se fait ensuite à 37°C.

Le marquage des fragments est opéré généralement à l'aide de quelques cycles d'amplification additionnels en présence d'oligonucléotides amorces présentant un marquage radioactif, enzymatique ou, de préférence, fluorescent, à base de fluorophores.

Le gel support de migration comprend généralement plusieurs pistes parallèles, par exemple 24 pistes.

Dans le cas préférentiel d'un marquage fluorescent à base de fluorophores, l'ensemble de détection comprend un système d'excitation (laser ou lampe halogène par exemple) et un capteur sensible au rayonnement fluorescent généré par l'échantillon excité. Dans certains dispositifs, un jeu de miroirs placés sur un chariot déplacé en translation en va et vient en regard de la base du gel, balaye successivement les diverses pistes, réfléchit le rayonnement d'excitation sur l'échantillon et renvoie le rayonnement fluorescent sur le capteur.

Dans d'autres dispositifs, le rayonnement d'excitation est directement envoyé dans l'épaisseur du gel, perpendiculairement à la direction d'électrophorèse et parallèlement au plan du gel, tandis qu'une série de capteurs recueille le signal de fluorescence.

Le capteur délivre un signal dont l'amplitude est proportionnelle à la concentration de la couleur fluorescente détectée, pourvu que cette amplitude reste inférieure à un certain seuil.

Par ailleurs, pour certains dispositifs, on sait utiliser simultanément quatre fluorophores de "couleurs" différentes, ce qui permet de placer quatre échantillons de marquages différents sur chaque piste.

Pour cela, il est simplement nécessaire de prévoir un ensemble de filtres motorisé par exemple une roue à quatre filtres déplacée séquentiellement, en amont du capteur, pour déceler successivement chacun des rayonnements et détecter par conséquent chacun des échantillons.

La présente invention peut par exemple trouver application dans le procédé de description des répertoires du système immunitaire, présenté dans la demande de brevet déposée en France le 9.01.1991 sous le N°91 00189.

Ce procédé se caractérise essentiellement en ce que

- à partir d'un prélèvement biologique, on effectue la transcription réverse des ARNm qu'il contient,
- on effectue ensuite, sur le produit de transcription (ou directement sur l'ADN extrait de l'échantillon), des amplifications séparées pour chaque couple d'amorce V, C, V correspondant à un segment variable du répertoire en cause et C s'hybridant au segment constant du repertoire étudié,
- sur chacun de ces produits d'amplification on effectue, pour chaque segment J du répertoire, marqué, une étape d'élongation utilisant comme amorce un oligonucléotide spécifique de ce segment J et le produit d'amplification comme matrice,
- pour chaque produit d'élongation correspondant à un triplet V,C,J ainsi obtenu on fait apparaitre la taille et la quantité des différents produits d'élongation,
- la description du répertoire est réalisée pour chaque élément du répertoire correspondant à un triplet V,C,J et à la taille de l'élément par la mesure de la quantité de cet élément dans ledit répertoire.

On se reportera utilement à la description de cette demande de brevet FR 91 00189 pour la bonne compréhension de ce processus de description des répertoires du système immunitaire.

La présente invention peut également trouver application dans le procédé de détermination de la quantité d'un fragment d'ADN d'intérêt par une méthode d'amplification quantitative, présenté dans la demande de brevet déposée en France le 15 Novembre 1991 sous le n° 91 14089.

Ce procédé se caractérise essentiellement en ce que :

1) on ajoute à l'échantillon à analyser contenant le fragment d'ADN d'intérêt, un fragment d'ADN standard différent du fragment d'ADN d'intérêt mais amplifiable par les mêmes oligonucléotides amorces, les fragments d'ADN standards et d'intérêt ne différant en séquences et/ou en taille de pas plus de 10% environ, de préférence de pas plus de 5 nucélotides par brin,
2) on co-amplifie les fragments d'ADN d'intérêt et standard avec les mêmes oligonucléotides amorces, de préférence jusqu'à saturation de l'amplification du mélange de ces fragments d'ADN,
3) on ajoute dans le milieu réactionnel obtenu à l'étape 2) un ou plusieurs oligonucléotide(s) amorce(s) marqué(s), spécifique(s) des fragments d'ADN d'intérêt et standards, et différents desdits oligonucléotides amorces de l'étape 2), et on effectue un ou quelques cycle(s) d'amplification supplémentaire(s) avec le ou lesdits oligonucléotide(s) amorce(s) marqué(s), de sorte que, au cours d'un cycle, après dénaturation de l'ADN, le ou lesdits oligonucléotide(s) amorce(s) marqué(s) s'hybride(nt) avec lesdits fragments en un site approprié pour qu'une élongation par l'ADN polyméase génère des fragments d'ADN marqués de taille et/ou de séquences différentes ou avec des marqueurs différents selon qu'ils proviennent des fragments d'ADN d'intérêt ou standards respectivement, puis
4) on détermine la quantité initiale de fragment d'ADN d'intérêt comme étant le produit de la quantité initiale de fragment d'ADN standard et du rapport entre la quantité de fragment d'ADN d'intérêt amplifié et la quantité de fragment d'ADN standard amplifié, rapport qui est identique à celui des quantités des fragments d'ADN marqués provenant respectivement des fragments d'ADN d'intérêt et standards amplifiés obtenus à l'étape 3).

Selon une mise en oeuvre particulière, la détermination de la dernière étape du procédé précité se fait en :

- séparant selon leur taille par électrophorèse sur gel les fragments d'ADN marqués provenant des fragments d'ADN d'intérêt et standards amplifiés, puis en
- détectant les intensités des signaux correspondant au marqueur d'une amorce respective pour les fragments d'ADN marqués provenant des fragments d'ADN d'intérêt et standards respectivement.

On se reportera utilement à la description de cette demande de brevet FR 91 14089 pour la bonne compréhension de ce processus de détermination de la quantité d'un fragment d'ADN d'intérêt.

Dans les appareillages connus, la taille en nucléotides des fragments d'ADN est évaluée de façon assez rudimentaire à l'aide d'une comparaison visuelle de la position des échantillons détectés par rapport à la position de standards de taille marqués qui sont codéposés sur le gel support et soumis à la même électrophorèse que les échantillons à mesurer.

La présente invention a pour but de perfectionner la technique existante en proposant de nouveaux moyens permettant une mesure plus précise de la taille des fragments d'ADN.

Ce but est atteint selon la présente invention grâce à un procédé comprenant les étapes qui consistent à :

i) mesurer le temps de migration, sur une longueur constante prédéterminée, de chaque fragment d'ADN détecté, et
ii) établir une corrélation entre la taille de chaque fragment d'ADN détecté et le temps de migration de celui-ci.

Selon une caractéristique avantageuse de la présente invention, la corrélation entre la taille de chaque fragment d'ADN détecté et le temps de migration de celui-ci, est établi sur la base de la loi :

$$L(t)=A \exp[-B/(t+to)]+C$$

dans laquelle A, B et C sont des constantes, t représente le temps de migration, to représente une constante de temps et L(t) représente la longueur en nucléotides de l'ADN détecté.

D'autres caractéristiques, buts et avantages de la présente invention apparaîtront à la lecture de la description détaillée qui va suivre, et en regard des dessins annexés donnés à titre d'exemple non limitatif et sur lesquels :

- la figure 1 représente un organigramme schématique général du procédé conforme à la présente invention,
- les figures 2 à 7 représentent sous forme d'organigrammes des étapes particulières de ce procédé, et
- la figure 8 représente un organigramme général regroupant l'ensemble des étapes qui vont être décrites par la suite.

Comme indiqué précédemment, le procédé conforme à la présente invention, conçu pour déterminer la taille en nucléotides de fragments d'ADN, exploite un processus de séparation des fragments d'ADN par électrophorèse sur gel.

Ce processus de séparation connu en lui-même et rappelé ci-dessus dans ses caractéristiques essentielles ne sera pas décrit plus en détail par la suite.

Plus précisément, la présente invention exploite un processus selon lequel des standards de taille connue sont codéposés sur le gel support. De préférence, il est ainsi déposé des standards de taille connue sur trois pistes témoins, à savoir sur les deux bords latéraux du gel support et au centre de celui-ci.

Le nombre n de standards de taille ainsi codéposés sur chaque piste témoin du gel support peut être choisi quelconque par l'utilisateur. Toutefois, de préférence, on dépose cinq standards de taille sur chaque piste témoin.

A titre d'exemple non limitatif, ces cinq standards de taille connue peuvent comprendre respectivement 96, 114, 140, 157 et 176 nucléotides.

Comme cela est schématisé sur la figure 1 annexée, le procédé conforme à la présente invention comprend de préférence les étapes consistant à :

a) opérer une électrophorèse sur gel d'échantillons d'ADN marqués et de standards de taille marqués codéposés sur le gel (étape 100 sur la figure 1),
b) détecter et mémoriser le signal issu du capteur de l'ensemble de détection (étape 200 sur la figure 1),
c) filtrer les données mesurées (étape 300 sur la figure 1),
d) déterminer la taille des fragments d'ADN détectés (étape 400 sur la figure 1), et
e) visualiser les résultats obtenus (étape 500 sur la figure 1).

Le cas échéant, l'étape de filtrage 300 peut être opérée avant l'étape de mémorisation 200.

De préférence, à l'étape 200, les courbes issues du capteur sont stockées dans un fichier spécifique respectif à raison d'un fichier par piste de mesure.

La phase de filtrage 300 comprend de préférence, comme cela est schématisé sur la figure 2, l'étape initiale 210 de filtrage passe-bas modulé par une convolution de Lanczos, afin d'éliminer le bruit haute fréquence, à l'aide d'un filtre présentant de préférence une fréquence de coupure réglable et une largeur de fenêtre réglable.

La largeur de la fenêtre de Lanczos est également réglable.

Enfin, la phase de filtrage 300 comprend l'étape ultime 320 de soustraction de la ligne de base obtenue en tout point, comme la valeur minimale de la courbe filtrée, sur une valeur de fenêtre déterminée.

La largeur de cette fenêtre doit bien sûr être bien supérieure à la largeur d'un pic ; de préférence, la largeur de cette fenêtre est réglable.

On va maintenant décrire l'étape 400 de détermination de la taille des fragments d'ADN détectés.

Comme cela a été évoqué précédemment, selon l'invention, l'étape principale de détermination de la taille des fragments d'ADN consiste, après avoir mesuré le temps de migration de chaque fragment d'ADN détecté, à établir une corrélation entre la taille L de chaque fragment d'ADN détecté et le temps de migration de celui-ci à l'aide de la relation :

$$L(t)= A \exp[-B/(t+to)]+C$$

dans laquelle A, B et C sont des constantes, t représente le temps de migration et to représente une constante de temps.

On notera que to=0 lorsque l'origine temporelle de la mesure coïncide avec le début de l'électrophorèse.

Plus précisément, comme cela est schématisé sur la figure 3, l'étape 400 consiste à :

a) déterminer les coefficients A, B, C pour chaque piste témoin comportant des standards de taille (étape 410 sur la figure 3), dont les temps de migration $t_i$ ont été mesurés,
b) opérer une interpolation des temps de migration $t_i$ mesurés dans les pistes témoin, pour les pistes de mesure (étape 420 sur la figure 3),
c) à partir de cette interpolation, déterminer, pour chaque piste de mesure la valeur des paramètres A, B et C (étape 430 sur la figure 3)
d) déterminer la taille des fragments d'ADN sur la base de la loi précitée (étape 440 sur la figure 3) établie pour chacune des pistes du gel.

L'étape 410 est illustrée sous forme d'organigramme sur la figure 4.

Cette étape 410 débute par une étape 411 de saisie des i standards de taille dans le fichier correspondant. Cette étape 411 détaillée sur la figure 5 est opérée comme suit.

Dans une première phase 4110, l'appareillage recherche automatiquement dans une fenêtre de mesure fixée par défaut, mais modifiable par l'utilisateur, les i pics de rayonnement. Cette recherche est opérée en comparant (étape 4111) les pics d'intensité mesurés avec un seuil progressivement abaissé (étape 4112) jusqu'à obtention des i pics (étape 4113).

Si la première phase 4110 ci-dessus ne permet pas d'obtenir les i pics recherchés, l'appareillage passe à une seconde phase 4114 dans laquelle l'utilisateur peut soit modifier la largeur de la fenêtre à l'étape 4115 et relancer la phase de recherche automatique précitée 4110, soit opérer une recherche manuelle (étape 4116) en identifiant par tous moyens connus (par exemple à l'aide d'un pointeur déplacé sur écran) les pics recherchés, sur une représentation de la courbe mesurée visualisée sur écran.

Comme cela est représenté sur la figure 4 annexée, une fois les standards de taille identifiée de façon automatique ou manuelle à l'étape 411 de saisie des standards, cette étape est suivie d'une étape 412 de mesure du temps ti de migration des standards de longueur connue Li puis d'une étape 413 de calcul des coefficients A, B et C pour ces standards sur la base de cette mesure.

Le temps de migration ti des standards de taille, mesuré à l'étape 412, correspond au temps mis par ceux-ci pour atteindre le capteur de l'ensemble de détection placé à la base d'un gel support, à partir d'une origine temporelle.

Cette origine temporelle peut correspondre à l'initialisation de l'électrophorèse, auquel cas la constante de temps to est nulle.

L'origine temporelle peut aussi être postérieure à l'initialisation de l'électrophorèse, auquel cas la constante de temps to est égale au décalage entre l'initialisation de l'électrophorèe et l'origine temporelle considérée. L'utilisateur introduit alors la valeur de ce paramètre une fois pour toute. Dans la suite, on prend $t_o = 0$ pour simplifier l'écriture.

L'étape 413 est illustrée sur la figure 6.

A l'étape 413, les coefficients A, B, C sont tout d'abord estimés (étape 4130) à l'aide de la fonction d'erreur suivante au moindres carrés :

$$f(A,B,C,) = \sum_{i=1}^{n} [L_i-(A\,\exp(-B/t_i)+C)]^2$$

dans laquelle n est le nombre d'ADN standards,
en recherchant la valeur de ces coefficients A, B et C pour lesquels les fonctions dérivées partielles en A, B, C, de cette fonction f(A, B, C) sont nulles.

On obtient ainsi un système de trois équations dont l'une en fonction seulement de B :

$$[\sum_{i=1}^{n} (L_i-\bar{L})\exp(-B/t_i)]\ x$$

$$[\sum_{i=1}^{n} (\exp(-B/t_i)-\overline{\exp(-B/t)})\exp(-B/t_i)1/t_i] =$$

$$[\sum_{i=1}^{n} (L_i-\bar{L})\exp(-B/t_i)1/t_i]\ x$$

$$[\sum_{i=1}^{n} (\exp(-B/t_i)-\overline{\exp(-B/t)})\exp(-B/t_i)]$$

$$A=[\sum_{i=1}^{n}(L_i-\bar{L})\exp(-B/t_i)]\ /$$

$$[\sum_{i=1}^{n}(\exp(-B/t_i)-\overline{\exp(-B/t)})\exp(-B/t_i)]$$

$$C=(1/n)\sum_{i=1}^{n} [L_i-A\,\exp(-B/t_i)]$$

avec comme notation que :

$$\overline{X(t)} = (1/n) \sum_{i=1}^{n} X(t_i) \text{ est la moyenne des } X(1), \ldots, X(n).$$

La première équation ci-dessus permet donc de déterminer la valeur de la constante B.

Et une fois la constante B obtenue, on obtient les constantes A et C sur la base des deux dernières équations ci-dessus.

Une fois les coefficients A, B et C obtenus à l'étape 4130 pour les diverses pistes standards, les valeurs A, B, C sont réportées à l'étape 4131 dans la fonction f(A,B,C) ou L(t)=A exp(-B/t)+C afin de vérifier pour chaque valeur de consigne connue Li d'un standard que l'écart entre la taille théorique ainsi mesurée et la valeur de consigne connue Li, est inférieur à un seuil donné égal à un nombre prédéterminé de bases (étape 4132).

Dans l'affirmative, le procédé conforme à la présente invention est poursuivi par l'étape d'interpolation 420.

Dans la négative, le calcul des coefficients A, B et C est repris, en vue d'une optimisation, après modification du seuil défini par un nombre prédéterminé de bases, par l'utilisateur à l'étape 4133.

L'étape d'interpolation 420 a pour but de définir les valeurs des temps de migration $t_i$ des marqueurs non standards pour chaque piste de mesure d'échantillons sur la base des valeurs mesurées pour les pistes standards. Cette étape d'interpolation 420 a pour but de réduire les erreurs de mesure dues à une dispersion des paramètres de migration selon la largeur du gel support.

Il peut s'agir d'une interpolation linéaire.

Toutefois, dans le cadre de la présente invention, on peut procéder à une interpolation de type parabolique, par exemple l'interpolation de Simpson. L'interpolation de type parabolique donne en effet un résultat plus précis que l'interpolation linéaire.

Enfin dans l'étape 420, on détermine pour chaque piste de mesure, les valeurs des coefficients A, B et C à partir des valeurs $t_i$ résultant de l'interpolation, et ce à l'aide des relations données précédemment.

En pratique, certains appareillages nécessitent que tous les échantillons ne soient pas chargés sur le gel simultanément. Le plus souvent les gels supports possèdent ainsi des pistes paires ayant une origine temporelle commune et des pistes impaires ayant également une origine temporelle commune, mais décalée dans le temps par rapport aux pistes paires.

Dans ce cas, des standards de taille sont codéposés sur le gel support respectivement avec les mêmes origines que les diverses pistes de mesure.

Il est donc nécessaire de calculer les coefficients A, B, C, pour les standards de taille présentant des origines différentes correspondant respectivement aux pistes paires et impaires et de procéder à des interpolations respectives pour les pistes de mesure paires et impaires respectivement.

En d'autres termes, les étapes 410 et 412 sont réitérées pour chaque origine de piste de mesure.

Une fois les coefficients A, B et C connus, et de préférence mémorisés pour chaque piste de mesure, on procède à l'étape 440.

Comme cela est illustré sur la figure 7, cette étape 440 se décompose en trois sous-étapes suivantes :

a) saisie des pics du signal de mesure correspondant aux fragments d'ADN (étape 441 sur la figure 7). Cette saisie peut être opérée automatiquement en comparant l'amplitude du signal de mesure avec un seuil. Ce seuil peut être réglable ainsi que la largeur de la fenêtre considérée. La saisie des pics utiles peut aussi être opérée manuellement. L'opérateur recherche alors manuellement les pics du signal correspondant aux fragments d'ADN, comme décrit précédemment et illustré sur la figure 5 pour la saisie des standards.

b) mesure à l'étape 442 du temps de migration des fragments d'ADN, comme décrit précédemment à l'étape 412 pour les standards, et enfin

c) calcul à l'étape 443 de la taille des différents fragments d'ADN sur la base de la relation :

$$L(t)=A\exp[-B/(t+to)]+C$$

Une fois les résultats entièrement calculés, la représentation de ceux-ci peut être visualisée sous toute forme appropriée à l'étape 500.

Par exemple, dans le cadre d'un procédé de description des répertoires du système immunitaire présenté dans la demande FR 91 00189, on peut illustrer les résultats sous forme d'une matrice tridimensionnelle dont deux axes correspondent aux amorces V et J, tandis que le troisième axe correspond à la valeur de taille des segments d'ADN détectés.

Les différents échantillons analysés qui correspondent à des lignes différentes d'une section J/Taille de la matrice correspondent à des amorces J respectives et différentes d'une ligne à l'autre. Or ces diverses amorces J présentent des rendements différents. Pour permettre de comparer les valeurs représentées d'une ligne à l'autre, il faut donc normaliser les valeurs mesurées, en fonction de rendement des amorces J, avant d'opérer la visualisation.

De même, les amorces V possèdent des rendements différents. Par conséquent, pour permettre de comparer directement les valeurs représentées dans les diverses sections J/Taille on procède de préférence à une normalisation des valeurs mesurées d'une section à l'autre, sur la base de mesures quantitatives réalisées selon le procédé décrit dans la demande de brevet FR 91 14089 relative à la détermination de la quantité d'un fragment d'ADN d'intérêt, avant de procéder à la représentation.

La présente invention permet d'améliorer nettement la précision de mesure de taille des fragments d'ADN.

En effet, alors que la comparaison grossière des fragments d'ADN migrant par électrophorèse, avec des standards de taille connue codéposés, selon l'état de la technique, permet au mieux de connaître la longueur des fragments d'ADN avec une précision de l'ordre de 1 à 2%, selon l'invention au contraire, la précision de la mesure est de l'ordre de 0,3%.

Dans le cas de l'utilisation simultanée de plusieurs fluorophores émettant à des longueurs d'ondes différentes, comme indiqué précédemment, il est préférable de compléter le procédé qui vient d'être décrit, par un processus initial d'élimination du recouvrement des couleurs. On sait en effet que dans le cas de multiples fluorophores, le signal fluorescent détecté, donne une contribution importante dans le canal de la couleur ad hoc, mais également, du à l'imperfection du filtre situé devant le capteur, des contributions, certes moins importantes, dans les trois autres canaux. Afin d'éliminer ces signaux parasites, les données enregistrées peuvent subir une opération qui consiste à multiplier la matrice de p lignes (p représentant le nombre de fluorophores utilisés) et de n points (où n est le nombre de mesures réalisées pendant l'électrophorèse) par une matrice carrée (pxp) caractéristique de l'appareil. Une fois cette transformation linéaire réalisée, les contributions parasites au signal d'une couleur sont éliminées presque totalement.

## Revendications

1. Procédé de détermination de la taille en nucléotides de fragments d'ADN séparés par électrophorèse sur gel caractérisé par le fait qu'il comprend les étapes qui consistent à :

   i) mesurer le temps de migration, sur une longueur constante prédéterminée, de chaque fragment d'ADN détecté, et
   ii) établir une corrélation entre la taille de chaque fragment d'ADN détecté et le temps de migration de celui-ci.

2. Procédé selon la revendication 1, caractérisé par le fait que la corrélation entre la taille (L) de chaque fragment d'ADN détecté et le temps de migration de celui-ci est établie sur la base de la loi :

$$L(t)=A\exp[-B/(t+to)]+C$$

dans laquelle A, B, C sont des constantes, t représente le temps de migration et to représente une constante de temps.

3. Procédé selon l'une des revendications 1 ou 2, caractérisé par le fait que la corrélation entre la taille (L) de chaque fragment d'ADN détecté et le temps de migration de celui-ci est établi sur la base de la loi :

$$L(t)=AeXp[-B/t]+C$$

dans laquelle A, B, C sont des constantes et t représente le temps de migration.

4. Procédé selon l'une des revendications 2 ou 3, dans lequel des standards de taille connue sont codéposés sur le gel, caractérisé par le fait que la valeur des coefficients A, B et C est recherchée, pour les standards de taille connue, lorsque les fonctions dérivées partielles en A, B et C de la fonction suivante sont nulles :

$$f(A,B,C,) = \sum_{i=1}^{n} [L_i-(A\exp(-B/t_i)-C)]^2$$

dans laquelle $L_i$ représente les longueurs connues des standards de taille déposés, $t_i$ représente le temps de migration de chacun de ces standards connus.

5. Procédé selon la revendication 4, caractérisé par le fait que les standards de taille connue sont saisis automatiquement par comparaison de la courbe de mesure détectée dans une fenêtre de mesure réglable, avec un seuil progressivement abaissé jusqu'à obtention des i pics recherchés.

6. Procédé selon la revendication 4, caractérisé par le fait que les standards de taille connue sont saisis manuellement.

7. Procédé selon l'une des revendications 4 à 6, caractérisé par le fait que les coefficients A, B et C sont déterminés sur la base des relations suivantes (a), (b) et (c) :

$$(a)\ [\sum_{i=1}^{n} (L_i-\bar{L})\exp(-B/t_i)] \times [\sum_{i=1}^{n} (\exp(-B/t_i)-\overline{\exp(-B/t)})\exp(-B/t_i)1/t_i] =$$

$$[\sum_{i=1}^{n} (L_i-\bar{L})\exp(-B/t_i)1/t_i] \times [\sum_{i=1}^{n} (\exp(-B/t_i)-\overline{\exp(-B/t)})\exp(-B/t_i)]$$

$$(b)\ A=[\sum_{i=1}^{n}(L_i-\bar{L})\exp(-B/t_i)] / [\sum_{i=1}^{n}(\exp(-B/t_i)-\overline{\exp(-B/t)})\exp(-B/t_i)]$$

$$(c)\ C=1/n \sum_{i=1}^{n} [L_i-A\exp(-B/t_i)]$$

avec la notation que $\overline{X(t)}$ désigne la moyenne de la fonction X sur les valeurs $t_i$ :

$$\overline{X(t)} = (1/n)\sum_{i=1}^{n} X(t_i)$$

8. Procédé selon l'une des revendications 4 à 7, caractérisé par le fait que les coefficients A, B et C sont ensuite reportés, pour chaque standard de taille considérée dans la relation :

$$L(t)=A\exp[-B/(t+to)]+C,$$

les valeurs de taille ainsi obtenues étant comparées aux valeurs de taille connue afin de reprendre le processus de calcul des coefficients A, B et C en vue d'une optimisation si l'erreur entre la taille mesurée et la taille connue des standards dépasse un seuil prédéterminé.

**9.** Procédé selon l'une des revendications 4 à 8, caractérisé par le fait qu'il comprend en outre l'étape consistant à déterminer les coefficients A, B et C pour chaque piste de mesure à l'aide d'une interpolation des positions $t_i$ mesurées pour les standards de taille connue, suivie d'un calcul des coefficients A, B et C à partir des valeurs $t_i$ résultant de l'interpolation.

**10.** Procédé selon la revendication 9, caractérisé par le fait que l'interpolation est une interpolation linéaire.

**11.** Procédé selon la revendication 9, caractérisé par le fait que l'interpolation est de type parabolique.

**12.** Procédé selon la revendication 11, caractérisé par le fait que l'interpolation est une interpolation de Simpson.

**13.** Procédé selon l'une des revendications 1 à 12, caractérisé par le fait que le gel support utilisé pour l'électrophorèse présente des séries de pistes de mesure présentant des origines temporelles respectivement décalées et que le procédé comprend les étapes consistant à calculer les coefficients A, B et C pour les standards de taille et à opérer ultérieurement une interpolation des positions $t_i$ obtenues à l'aide des standards de taille, suivie d'un calcul des coefficients A, B et C correspondants, sur les pistes de mesure, pour chaque origine temporelle de migration.

**14.** Procédé selon l'une des revendications 1 à 13, caractérisé par le fait que les courbes issues du capteur sont mémorisées, de préférence à raison d'un fichier par piste de mesure.

**15.** Procédé selon l'une des revendications 1 à 14, caractérisé par le fait que les courbes issues du capteur font l'objet d'un filtrage.

**16.** Procédé selon la revendication 15, caractérisé par le fait que l'étape de filtrage comprend un filtrage passe-bas.

**17.** Procédé selon l'une des revendications 15 ou 16, caractérisé par le fait que l'étape de filtrage comprend un filtrage de Lanczos.

**18.** Procédé selon l'une des revendications 15 à 17, caractérisé par le fait qu'il comprend en outre l'étape de soustraction de la ligne de base de la courbe mesurée.

**19.** Procédé selon l'une des revendications 1 à 18, caractérisé par le fait qu'il est mis en oeuvre dans le cadre d'un procédé de description des répertoires du système immunitaire et qu'il comprend la visualisation du répertoire sous forme d'une matrice tridimensionnelle dont deux axes correspondent aux amorces V et J tandis que le troisième axe correspond à la valeur de taille des segments d'ADN détectés, et qu'il comprend en outre l'étape de normalisation des valeurs mesurées, en fonction du rendement des amorces J, avant d'opérer la visualisation sur chaque section J/Taille de la matrice.

**20.** Procédé selon la revendication 19, caractérisé par le fait qu'il comprend en outre l'étape de normalisation des valeurs mesurées, d'une section J/Taille à l'autre, avant de procéder à la représentation de la matrice.

**21.** Procédé selon l'une des revendications 1 à 20, caractérisé par le fait que les pics du signal correspondant aux fragments d'ADN sont saisis manuellement.

**22.** Procédé selon l'une des revendications 1 à 20, caractérisé par le fait que les pics du signal correspondant aux fragments d'ADN sont saisis automatiquement.

**Patentansprüche**

**1.** Verfahren zur Bestimmung der Größe in Nucleotiden von DNA-Fragmenten, die durch Elektrophorese auf einem

Gel voneinander getrennt worden sind, dadurch gekennzeichnet, daß es die folgenden Stufen umfaßt:

i) Messung der Wanderungszeit (Laufzeit) jedes nachgewiesenen DNA-Fragments über eine vorgegebene konstante Länge (Strecke) und
ii) Aufstellung einer Korrelation zwischen der Größe jedes nachgewiesenen DNA-Fragments und der Wanderungszeit (Laufzeit) desselben.

**2.** Verfahren nach Anspruch 1, dadurch gekennzeichnet, daß die Korrelation zwischen der Größe (L) jedes nachgewiesenen DNA-Fragments und der Wanderungszeit desselben aufgestellt wird auf der Grundlage des Gesetzes:

$$L(t)=A\exp[-B/(t+to)]+C$$

worin A, B, C Konstanten sind, t die Wanderungszeit (Laufzeit) und to eine Zeitkonstante darstellen.

**3.** Verfahren nach einem der Ansprüche 1 oder 2, dadurch gekennzeichnet, daß die Korrelation zwischen der Größe (L) jedes nachgewiesenen DNA-Fragments und der Wanderungszeit (Laufzeit) desselben aufgestellt wird auf der Basis des Gesetzes:

$$L(t)=A\exp[-B/t]+C$$

worin A, B, C Konstanten sind und t die Wanderungszeit (Laufzeit) darstellt.

**4.** Verfahren nach einem der Ansprüche 2 oder 3, bei dem gleichzeitig bekannte Größenstandards auf das Gel aufgegeben (abgeschieden) werden, dadurch gekennzeichnet, daß der Wert der Koeffizienten A, B und C für die bekannten Größenstandards ermittelt wird, wenn die abgeleiteten Partialfunktionen A, B und C der nachfolgenden Funktion Null sind

$$f(A,B,C,) = \sum_{i=1}^{n}[L_i-(A\exp(-B/t_i)-C)]^2$$

in der $L_i$ die bekannten Längen (Strecken) der aufgegebenen (abgeschiedenen) Größenstandards und $t_i$ die Wanderungszeit (Laufzeit) jedes dieser bekannten Standards darstellen.

**5.** Verfahren nach Anspruch 4, dadurch gekennzeichnet, daß die bekannten Größenstandards automatisch erfaßt werden durch Vergleich der ermittelten Meßkurve in einem regulierbaren Meßfenster mit einem zunehmend niedrigeren Schwellenwert, bis die gewünschten i-Peaks erhalten werden.

**6.** Verfahren nach Anspruch 4, dadurch gekennzeichnet, daß die bekannten Größenstandards manuell erfaßt werden.

**7.** Verfahren nach einem der Ansprüche 4 bis 6, dadurch gekennzeichnet, daß die Koeffizienten A, B und C auf der Basis der folgenden Beziehungen (a), (b) und (c) bestimmt werden:

$$(a)\left[\sum_{i=1}^{n}(L_i-\bar{L})\exp(-B/t_i)\right] \times \left[\sum_{i=1}^{n}(\exp(-B/t_i)-\overline{\exp(-B/t)})\exp(-B/t_i)\,1/t_i\right] =$$

$$[\sum_{i=1}^{n}(L_i-\bar{L})\exp(-B/t_i)1/t_i] \times [\sum_{i=1}^{n}(\exp(-B/t_i)-\overline{\exp(-B/t)})\exp(-B/t_i)]$$

$$(b)\ A=[\sum_{i=1}^{n}(L_i-\bar{L})\exp(-B/t_i)] / [\sum_{i=1}^{n}(\exp(-B/t_i)-\overline{\exp(-B/t)})\exp(-B/t_i)]$$

$$(c)\ C=1/n\sum_{i=1}^{n}[L_i-A\exp(-B/t_i)]$$

wobei $\overline{X(t)}$ den Mittelwert der Funktion X über die Werte $t_i$ darstellt:

$$\overline{X(t)}=(1/n)\sum_{i=1}^{n}X(t_i)$$

**8.** Verfahren nach einem der Ansprüche 4 bis 7, dadurch gekennzeichnet, daß die Koeffizienten A, B und C anschließend für jeden in Betracht gezogenen Größenstandard in die Beziehung

$$L(t)=A\exp[-B/(t+to)]+C$$

eingesetzt werden, wobei die so erhaltenen Größenwerte mit den bekannten Größenwerten verglichen werden, um den Prozeß der Berechnung der Koeffizienten A, B und C wieder aufzunehmen zur Erzielung einer Optimierung, wenn der Fehler zwischen der gemessenen Größe und der bekannten Größe der Standards einen vorgegegebenen Schwellenwert übersteigt.

**9.** Verfahren nach einem der Ansprüche 4 bis 8, dadurch gekennzeichnet, daß es außerdem eine Stufe umfaßt, bei der die Koeffizienten A, B und C für jede Meßstrecke mittels einer Interpolation der für die bekannten Größenstandards gemessenen Positionen $t_i$ bestimmt werden, woran sich eine Berechnung der Koeffizienten A, B und C aus den aus der Interpolation resultierenden Werten $t_i$ anschließt.

**10.** Verfahren nach Anspruch 9, dadurch gekennzeichnet, daß es sich bei der Interpolation um eine lineare Interpolation handelt.

**11.** Verfahren nach Anspruch 9, dadurch gekennzeichnet, daß die Interpolation eine solche vom Parabel-Typ ist.

**12.** Verfahren nach Anspruch 11, dadurch gekennzeichnet, daß die Interpolation eine Simpson-Interpolation ist.

**13.** Verfahren nach einem der Ansprüche 1 bis 12, dadurch gekennzeichnet, daß das für die Elektrophorese verwendete Trägergel Reihen von Meßstrecken aufweist, die jeweils zeitlich versetzte Aufgabe-punkte darstellen, und daß das Verfahren Stufen umfaßt, die darin bestehen, daß die Koeffizienten A, B und C für die Größenstandards berechnet werden und schließlich eine Interpolation der mit Hilfe der Größenstandards erhaltenen Positionen $t_i$ durchgeführt wird, gefolgt von einer Berechnung der entsprechenden Koeffizienten A, B und C über die Meßstrekken für jeden zeitlichen Aufgabepunkt der Wanderung.

**14.** Verfahren nach einem der Ansprüche 1 bis 13, dadurch gekennzeichnet, daß die von dem Meßfühler angegebenen Kurven gespeichert werden, vorzugsweise in einer Kartei pro Meßstrecke.

**15.** Verfahren nach einem der Ansprüche 1 bis 14, dadurch gekennzeichnet, daß die von dem Meßfühler angegebenen Kurven Gegenstand einer Filtration sind.

**16.** Verfahren nach Anspruch 15, dadurch gekennzeichnet, daß die Filtration eine Tiefpass-Filtration umfaßt.

**17.** Verfahren nach einem der Ansprüche 15 oder 16, dadurch gekennzeichnet, daß die Filtrationsstufe eine Lanczos-Filtration umfaßt.

**18.** Verfahren nach einem der Ansprüche 15 bis 17, dadurch gekennzeichnet, daß es außerdem umfaßt die Subtraktion der Basislinie der gemessenen Kurve.

**19.** Verfahren nach einem der Ansprüche 1 bis 18, dadurch gekennzeichnet, daß es im Rahmen eines Verfahrens zur Beschreibung der Repertoire des Immunsystems angewendet wird und daß es die Sichtbarmachung des Repertoirs in Form einer dreidimensionalen Matrix umfaßt, bei der zwei Achsen den Startern V und J entsprechen, während die dritte Achse dem Wert der Größe der nachgewiesenen DNA-Segmente entspricht, und daß es außerdem eine Normierung der gemessenen Werte als Funktion der Ausbeute der Starter J vor dem Sichtbarmachen auf jedem Abschnitt J/Größe der Matrix umfaßt.

**20.** Verfahren nach Anspruch 19, dadurch gekennzeichnet, daß es außerdem eine Normierung der gemessenen Werte, von einem Abschnitt J/Größe zum anderen umfaßt, bevor die Matrix dargestellt wird.

**21.** Verfahren nach einem der Ansprüche 1 bis 20, dadurch gekennzeichnet, daß die Peaks des den DNA-Fragmenten entsprechenden Signals manuell erfaßt werden.

**22.** Verfahren nach einem der Ansprüche 1 bis 20, dadurch gekennzeichnet, daß die Peaks des den DNA-Fragmenten entsprechenden Signals automatisch erfaßt werden.

## Claims

**1.** Method for determining the nucleotide size of DNA fragments separated by gel electrophoresis, characterized by the fact that it comprises the stages which consist in:

i) measuring the migration time, over a predetermined constant length, for each detected DNA fragment, and
ii) correlating the size of each detected DNA fragment with its migration time.

**2.** Method according to Claim 1, characterized by the fact that the correlation between the size (L) of each detected DNA fragment and its migration time is established based on the law:

$$L(t)=A\exp[-B/(t+to)]+C$$

in which A, B, C are constants, t represents the migration time and to represents a time constant.

**3.** Process according to either of Claims 1 and 2, characterized by the fact that the correlation between the size (L) of each detected DNA fragment and its migration time is established based on the law:

$$L(t)=A\exp[-B/t]+C$$

in which A, B, C are constants and t represents the migration time.

**4.** Method according to either of Claims 2 and 3, in which the standards of known size are codeposited on the gel, characterized by the fact that the value of the coefficients A, B, and C is evaluated, for the standards of known size, when the partial derivatives for A, B and C of the following function are zero:

$$f(A,B,C) = \sum_{i=1}^{n} [L_i-(A\exp(-B/t_i)-C)]^2$$

in which $L_i$ represents the known lengths of the deposited size standards, $t_i$ represents the migration time of each of these known standards.

5. Method according to Claim 4, characterized by the fact that the standards of known size are automatically acquired by comparing the measurement curve detected in an adjustable measurement window with a threshold which is gradually reduced until the desired i peaks are obtained.

6. Method according to Claim 4, characterized by the fact that the standards of known size are acquired manually.

7. Method according to one of Claims 4 to 6, characterized by the fact that the coefficients A, B and C are determined based on the following equations (a), (b) and (c) :

$$(a) [\sum_{i=1}^{n} (L_i-\bar{L})\exp(-B/t_i)] \times [\sum_{i=1}^{n} (\exp(-B/t_i)-\overline{\exp(-B/t)})\exp(-B/t_i)1/t_i] =$$

$$[\sum_{i=1}^{n} (L_i-\bar{L})\exp(-B/t_i)1/t_i] \times [\sum_{i=1}^{n} (\exp(-B/t_i)-\overline{\exp(-B/t)})\exp(-B/t_i)]$$

$$(b)\ A=[\sum_{i=1}^{n}(L_i-\bar{L})\exp(-B/t_i)] / [\sum_{i=1}^{n}(\exp(-B/t_i)-\overline{\exp(-B/t)})\exp(-B/t_i)]$$

$$(c)\ C=1/n \sum_{i=1}^{n} [L_i-A\exp(-B/t_i)]$$

with the notation that $\overline{X(t)}$ designates the mean of the function X over the $t_i$ values:

$$\overline{X(t)} = (1/n) \sum_{i=1}^{n} X(t_i)$$

8. Method according to one of Claims 4 to 7, characterized by the fact that the coefficients A, B and C are then applied, for each considered size standard, into the equation:

$$L(t)=A\exp[-B/(t+to)]+C,$$

the size values thus obtained being compared with the known size values so as to continue the process for calculating the coefficients A, B and C, for the purpose of optimization, if the error between the measured size and the known size of the standards exceeds a predetermined threshold.

**9.** Method according to one of Claims 4 to 8, characterized by the fact that it comprises, in addition, the stage consisting in determining the coefficients A, B and C for each measurement lane by means of an interpolation of the $t_i$ positions measured for the standards of known size, followed by calculation of the coefficients A, B and C from the $t_i$ values resulting from the interpolation.

**10.** Method according to Claim 9, characterized by the fact that the interpolation is a linear interpolation.

**11.** Method according to Claim 9, characterized by the fact that the interpolation is of the parabolic type.

**12.** Method according to Claim 11, characterized by the fact that the interpolation is a Simpson interpolation.

**13.** Method according to one of Claims 1 to 12, characterized by the fact that the supporting gel used for the electrophoresis has series of measurement lanes having time origins which are respectively separated and in that the method comprises the stages consisting in calculating the coefficients A, B and C for the size standards and in subsequently carrying out an interpolation of the $t_i$ positions obtained with the aid of the size standards, followed by a calculation of the corresponding coefficients A, B and C, on the measurement lanes, for each time origin of migration.

**14.** Method according to one of Claims 1 to 13, characterized by the fact that the curves derived from the sensor are stored, preferably at the rate of one file per measurement lane.

**15.** Method according to one of Claims 1 to 14, characterized by the fact that the curves derived from the sensor are subjected to filtering.

**16.** Method according to Claim 15, characterized by the fact that the filtering stage comprises a low-pass filtering.

**17.** Method according to either of Claims 15 and 16, characterized by the fact that the filtering stage comprises a Lanczos filtering.

**18.** Method according to one of Claims 15 to 17, characterized by the fact that it comprises, in addition, the stage for subtraction of the base line from the measured curve.

**19.** Method according to one of Claims 1 to 18, characterized by the fact that it is carried out within the framework of a method for describing the repertoires of the immune system and in that it comprises the visualization of the repertoire in the form of a three-dimensional matrix of which two axes correspond to the V and J primers whereas the third axis corresponds to the value of the size of the DNA segments detected, and in that it comprises, in addition, the stage for standardizing the values measured, as a function of the yield of the J primers, before carrying out the visualization on each section J/Size of the matrix.

**20.** Method according to Claim 19, characterized by the fact that it comprises, in addition, the stage for standardizing the values measured, from one section J/Size to another, before carrying out the representation of the matrix.

**21.** Method according to one of Claims 1 to 20, characterized by the fact that the peaks of the signal corresponding to the DNA fragments are acquired manually.

**22.** Method according to one of Claims 1 to 20, characterized by the fact that the peaks of the signal corresponding to the DNA fragments are acquired automatically.

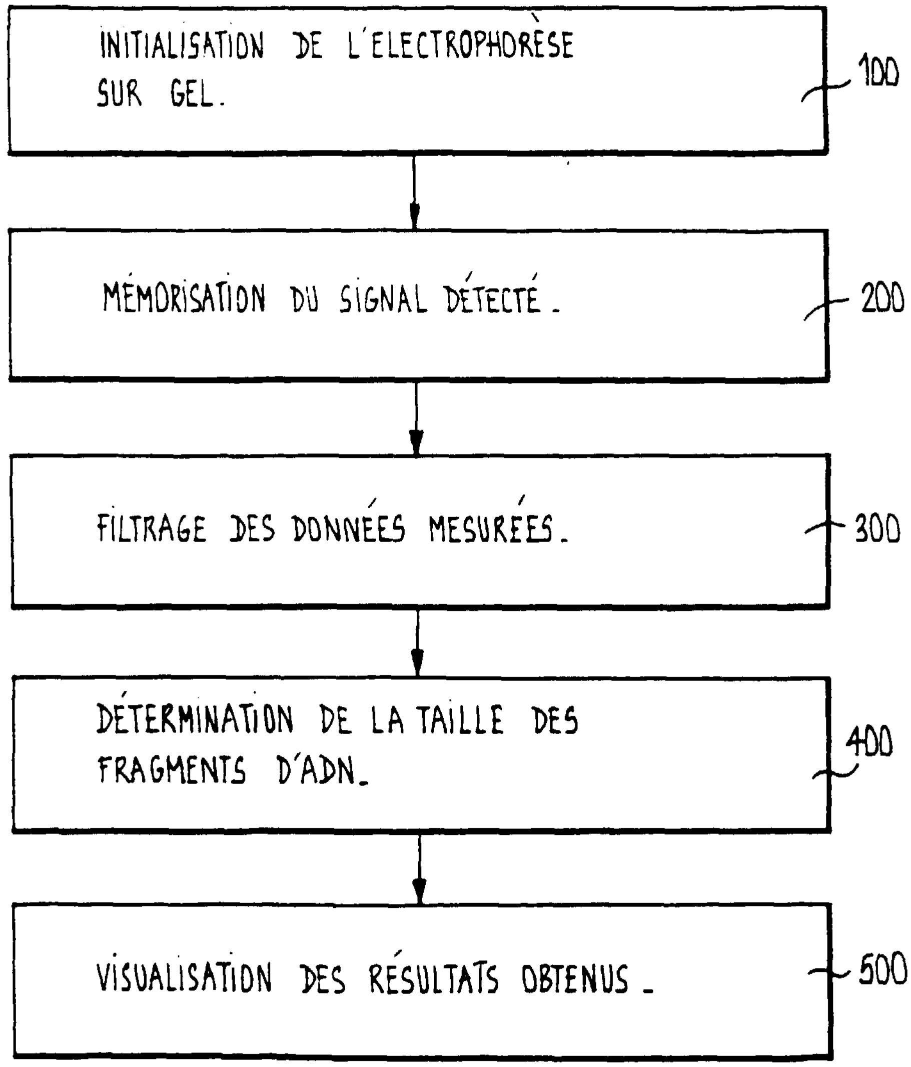
INITIALISATION DE L'ÉLECTROPHORÈSE SUR GEL.
100
MÉMORISATION DU SIGNAL DÉTECTÉ.
200
FILTRAGE DES DONNÉES MESURÉES.
300
DÉTERMINATION DE LA TAILLE DES FRAGMENTS D'ADN.
400
VISUALISATION DES RÉSULTATS OBTENUS.
500

FIG.1

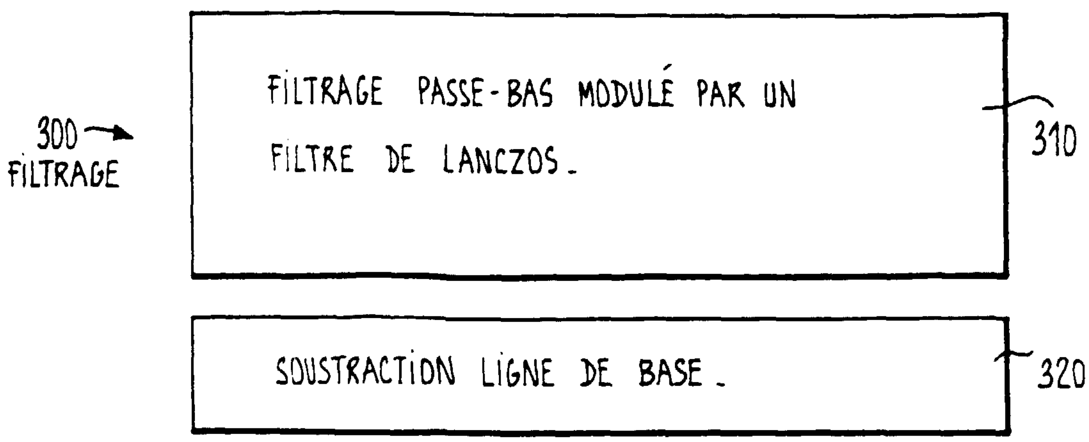
300
FILTRAGE
FILTRAGE PASSE-BAS MODULÉ PAR UN FILTRE DE LANCZOS.
310
SOUSTRACTION LIGNE DE BASE.
320

FIG.2

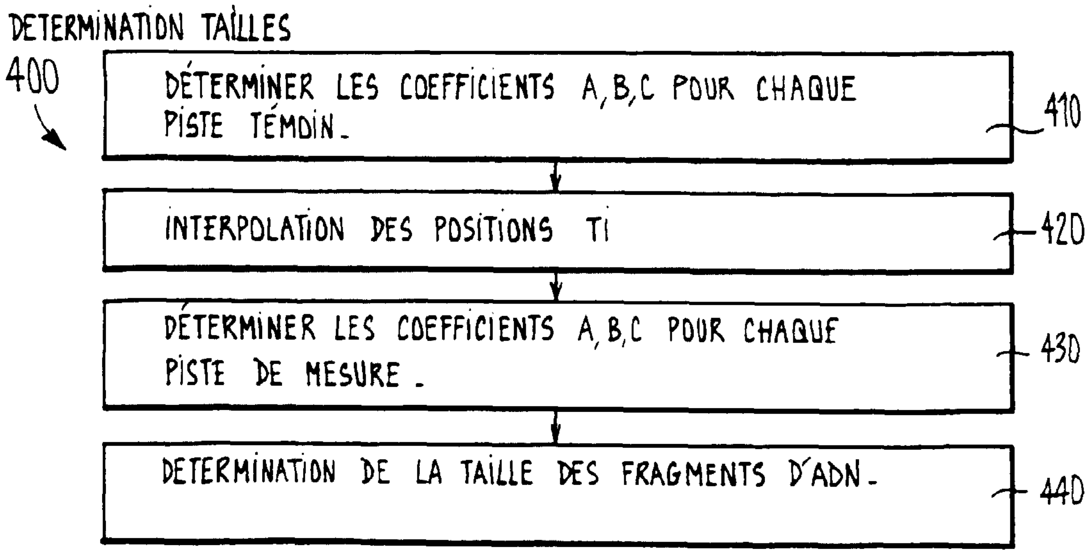


FIG. 3

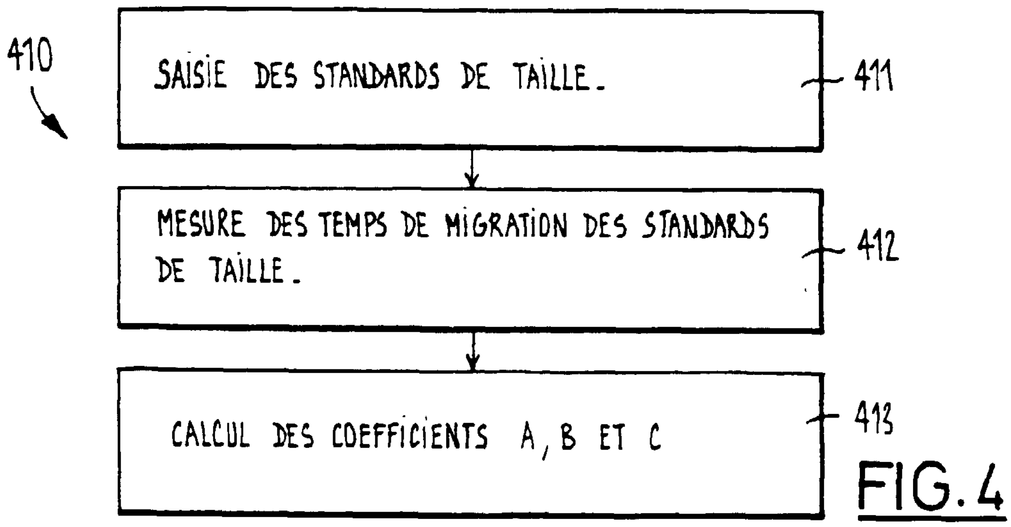


FIG. 4

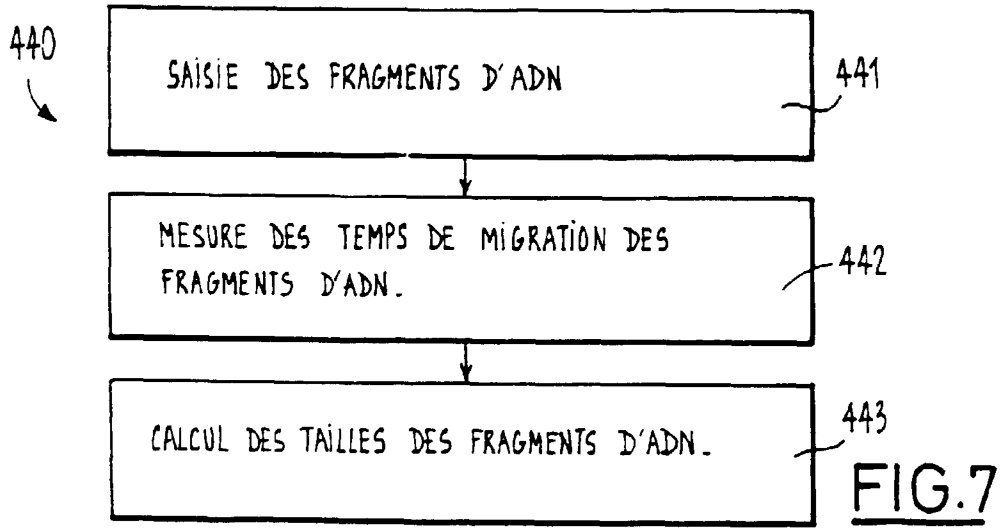


FIG. 7

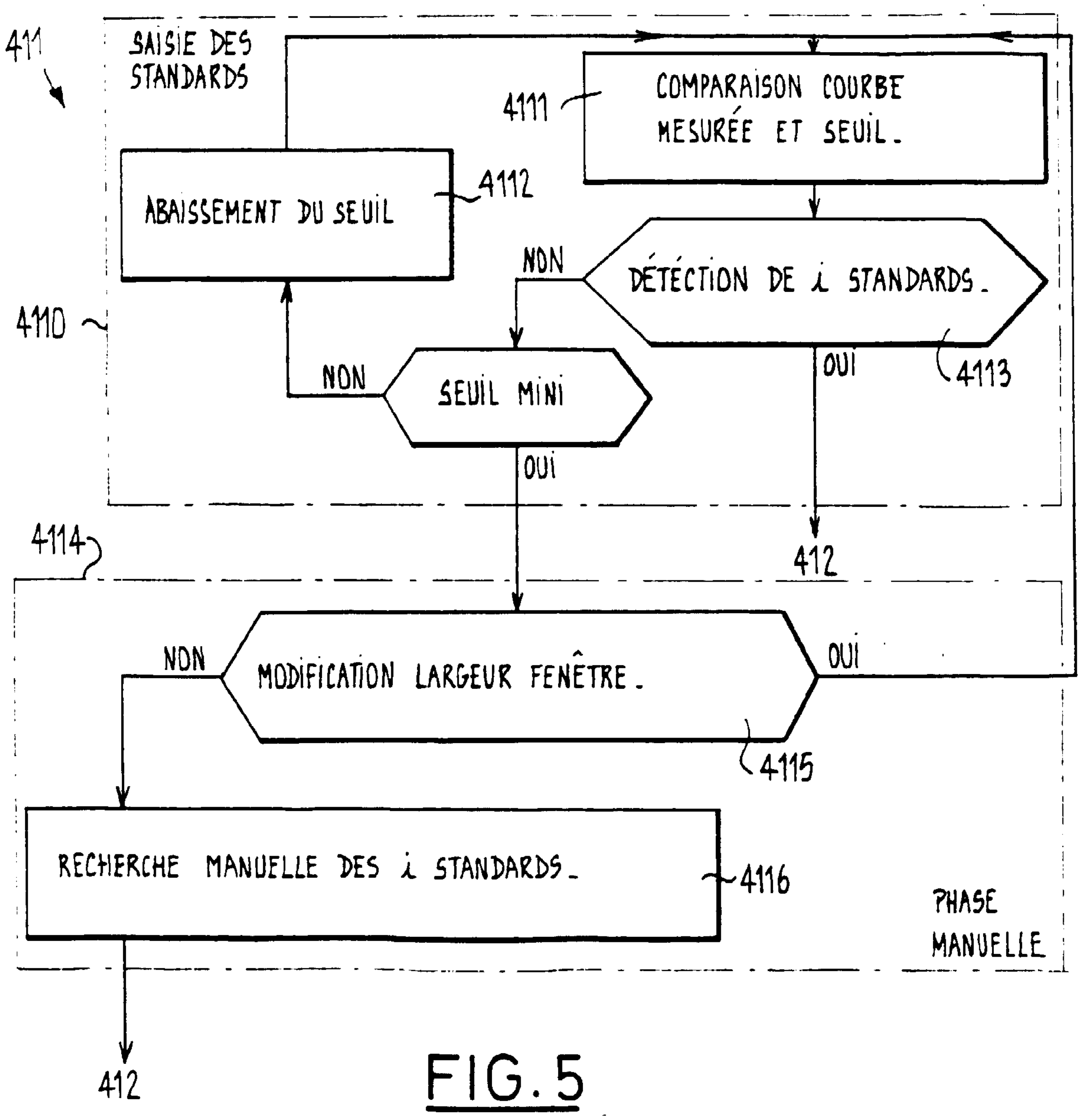
411
SAISIE DES STANDARDS
4111
COMPARAISON COURBE MESURÉE ET SEUIL.
ABAISSEMENT DU SEUIL
4112
NON
DÉTECTION DE i STANDARDS.
4110
NON
SEUIL MINI
OUI
4113
OUI
4114
412
NON
MODIFICATION LARGEUR FENÊTRE.
OUI
4115
RECHERCHE MANUELLE DES i STANDARDS.
4116
PHASE MANUELLE
412

FIG. 5

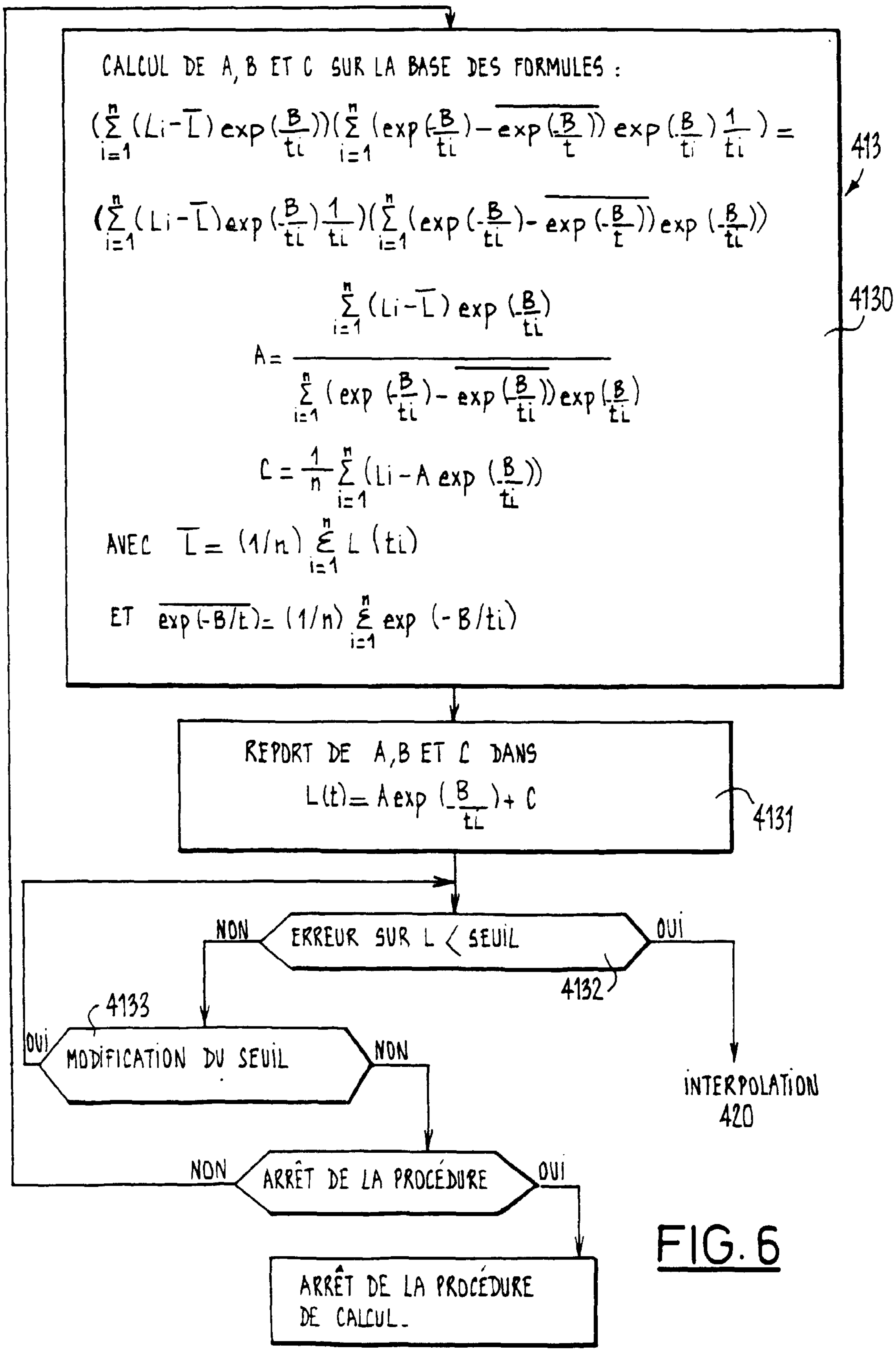
CALCUL DE A, B ET C SUR LA BASE DES FORMULES :
$\left(\sum_{i=1}^{n}(L_i-\overline{L})\exp\left(-\frac{B}{t_i}\right)\right)\left(\sum_{i=1}^{n}\left(\exp\left(-\frac{B}{t_i}\right)-\overline{\exp\left(-\frac{B}{t}\right)}\right)\exp\left(-\frac{B}{t_i}\right)\frac{1}{t_i}\right)=$
$\left(\sum_{i=1}^{n}(L_i-\overline{L})\exp\left(-\frac{B}{t_i}\right)\frac{1}{t_i}\right)\left(\sum_{i=1}^{n}\left(\exp\left(-\frac{B}{t_i}\right)-\overline{\exp\left(-\frac{B}{t}\right)}\right)\exp\left(-\frac{B}{t_i}\right)\right)$
$A=\frac{\sum_{i=1}^{n}(L_i-\overline{L})\exp\left(-\frac{B}{t_i}\right)}{\sum_{i=1}^{n}\left(\exp\left(-\frac{B}{t_i}\right)-\overline{\exp\left(-\frac{B}{t_i}\right)}\right)\exp\left(-\frac{B}{t_i}\right)}$
$C=\frac{1}{n}\sum_{i=1}^{n}\left(L_i-A\exp\left(-\frac{B}{t_i}\right)\right)$
AVEC $\overline{L}=(1/n)\sum_{i=1}^{n}L(t_i)$
ET $\overline{\exp(-B/t)}=(1/n)\sum_{i=1}^{n}\exp(-B/t_i)$
413
4130
REPORT DE A, B ET C DANS
$L(t)=A\exp\left(-\frac{B}{t_i}\right)+C$
4131
ERREUR SUR L < SEUIL
NON
OUI
4132
4133
MODIFICATION DU SEUIL
OUI
NON
INTERPOLATION
420
ARRÊT DE LA PROCÉDURE
NON
OUI
ARRÊT DE LA PROCÉDURE
DE CALCUL.

FIG. 6

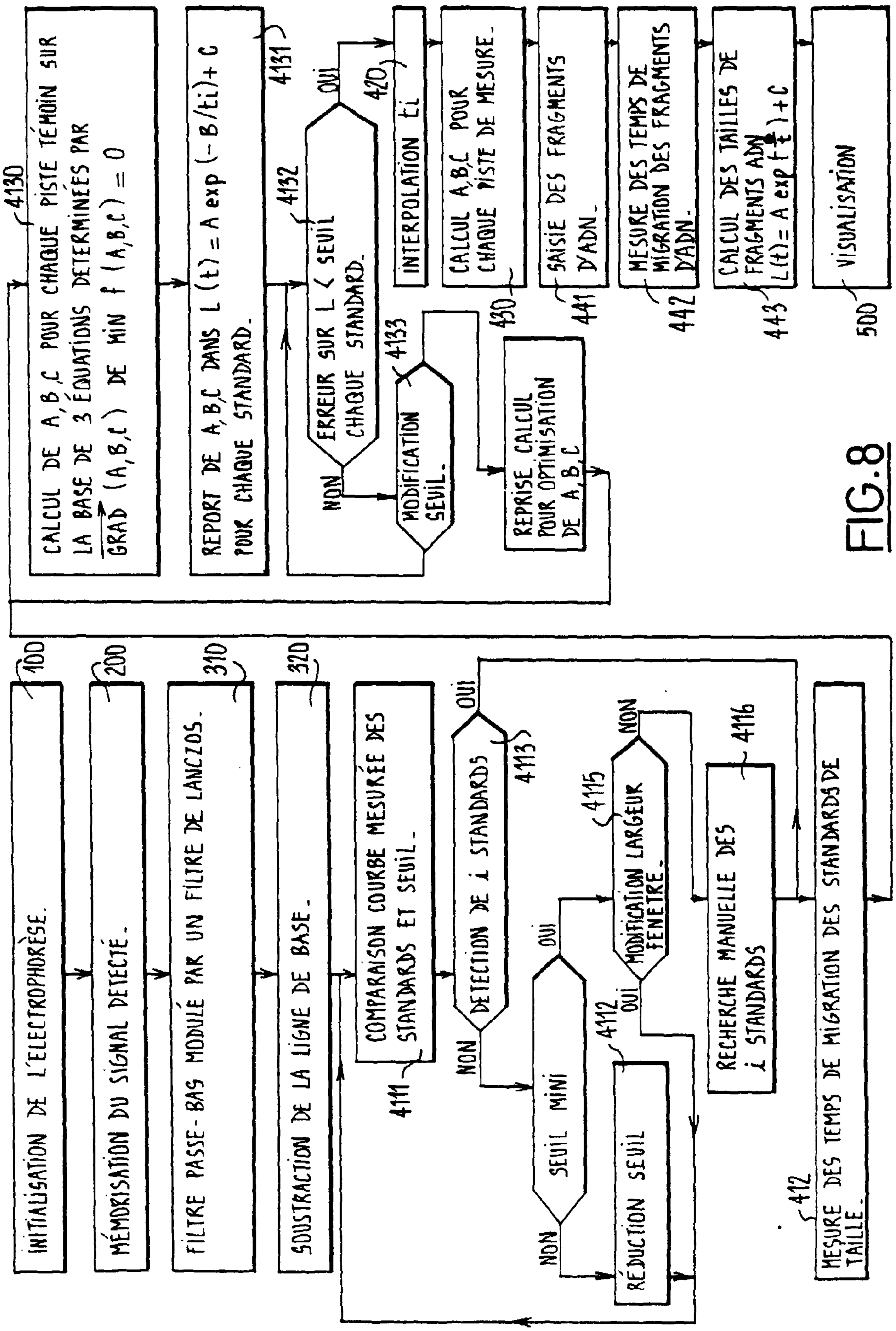
INITIALISATION DE L'ELECTROPHORÈSE.
100
MÉMORISATION DU SIGNAL DETECTÉ.
200
FILTRE PASSE-BAS MODULÉ PAR UN FILTRE DE LANCZOS.
310
SOUSTRACTION DE LA LIGNE DE BASE.
320
COMPARAISON COURBE MESURÉE DES STANDARDS ET SEUIL.
4111
DETECTION DE i STANDARDS
4113
OUI
NON
SEUIL MINI
OUI
NON
4112
RÉDUCTION SEUIL
MODIFICATION LARGEUR FENETRE.
4115
OUI
NON
RECHERCHE MANUELLE DES i STANDARDS
4116
MESURE DES TEMPS DE MIGRATION DES STANDARDS DE TAILLE.
412
CALCUL DE A,B,C POUR CHAQUE PISTE TÉMOIN SUR LA BASE DE 3 ÉQUATIONS DETERMINÉES PAR GRAD (A,B,C) DE MIN f (A,B,C) = 0
4130
REPORT DE A,B,C DANS L(t) = A exp (-B/ti) + C POUR CHAQUE STANDARD.
4131
ERREUR SUR L < SEUIL CHAQUE STANDARD.
4132
OUI
NON
MODIFICATION SEUIL.
4133
REPRISE CALCUL POUR OPTIMISATION DE A,B,C
INTERPOLATION ti
420
CALCUL A,B,C POUR CHAQUE PISTE DE MESURE.
430
SAISIE DES FRAGMENTS D'ADN.
441
MESURE DES TEMPS DE MIGRATION DES FRAGMENTS D'ADN.
442
CALCUL DES TAILLES DE FRAGMENTS ADN L(t) = A exp (-B/t) + C
443
VISUALISATION
500
FIG.8